(19) Europäisches Patentamt
European Patent Office
Office européen des brevets

(11) Publication number: **0 601 184 A1**

(12) **EUROPEAN PATENT APPLICATION published in accordance with Art. 158(3) EPC**

(21) Application number: **92917989.3**

(22) Date of filing: **24.08.92**

(86) International application number: **PCT/JP92/01067**

(87) International publication number: **WO 93/04069 (04.03.93 93/06)**

(51) Int. Cl.5: **C07D 495/04**, A61K 31/50

(30) Priority: **27.08.91 JP 240378/91**

(43) Date of publication of application: **15.06.94 Bulletin 94/24**

(84) Designated Contracting States: **AT BE CH DE DK ES FR GB GR IT LI LU MC NL SE**

(71) Applicant: **YOSHITOMI PHARMACEUTICAL INDUSTRIES, LTD.**
**6-9, Hiranomachi 2-chome**
**Chuo-ku**
**Osaka-shi Osaka 541(JP)**

(72) Inventor: **NAKAO, Tohru, Yoshitomi Pharmaceutical Ind. Ltd.**
**Cent. Res. Lab.,**
**955, Oaza-Koiwai,**
**Yoshitomimachi**
**Chikujo-gun, Fukuoka 871(JP)**
Inventor: **TANAKA, Hiroshi, Yoshitomi Pharmaceutical Ind. Ltd**
**Cent. Res. Lab.,**
**955, Oaza-Koiwai,**
**Yoshitomimachi**
**Chikujo-gun, Fukuoka 871(JP)**
Inventor: **MORIMOTO, Yasuto, Yoshitomi Pharmaceutical Ind Ltd**
**6-9, Hiranomachi 2-chome,**
**Chuo-ku**
**Osaka-shi, Osaka 541(JP)**
Inventor: **TAKEHARA, Shuzo, Yoshitomi Pharmaceutical Ind. Ltd**
**Cent. Res. Lab.,**
**955, Oaza-Koiwai,**
**Yoshitomimachi**
**Chikujo-gun, Fukuoka 871(JP)**

(74) Representative: **Selting, Günther et al**
**Patentanwälte**
**von Kreisler-Selting-Werner,**
**Bahnhofsvorplatz 1 (Deichmannhaus)**
**D-50667 Köln (DE)**

(54) **FUSED PYRIDAZINE COMPOUND AND PHARMACEUTICAL USE THEREOF.**

(57) A fused pyridazine compound represented by the general formula (I) and an antianxiety drug containing the same as the active ingredient: wherein $R^1$ represents carboxyl, formyl, cyano, hydroxy-iminomethyl, acyl, etc.; $R^2$ represents hydrogen, $C_1$ to $C_8$ alkyl, hydroxyalkyl, alkanoyloxyalkyl, aryl, aralkyl, heteroaryl, etc.; A represents sulfur or -CH=CH-; E represents $-CH_2-$ or $-S(O)_m-$, wherein m represents 0, 1 or 2; n represents 1 or 2; and the bond represented by both solid and broken lines is either a single or a double bond; provided that when A represents sulfur, E represents $-CH_2-$, while when A represents -CH=CH-, E represents $-S(O)_m-$, wherein m represents 0, 1 or 2, and n represents 2. This compound is reduced in side effects such as muscular relaxation, anesthesia potentiation, sedation, sleep/alcohol potentiation, etc., thus being useful as an antianxiety drug which selectively acts against anxiety.

EP 0 601 184 A1

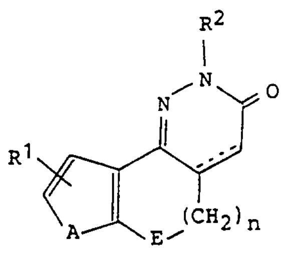

(I)

[Technical Field]

The present invention relates to fused pyridazine compounds useful as anxioselective anxiolytic agent, and pharmaceutical use thereof.

[Background Art]

Benzodiazepine (BZP) derivatives represented by diazepam have been used as, for example, anxiolytic agents. BZP agents exhibit excellent anxiolytic activity in human beings, whereas they are known to cause undesirable adverse reactions (side effects) such as sedation, muscle-relaxation and interaction with alcohol/barbiturates. Therefore, the development of anxioselective anxiolytic agents with less side effects than conventional BZP derivatives has been desired.

On the other hand, it has been proved that receptors which have specific affinity for BZP derivatives exist in the central nervous system by a late pharmacological study [Science, vol. 198, 848 (1977)]. The findings of BZP receptors in the central nervous system have remarkably advanced the research on the mechanism of BZP agents. It was suggested that the BZP receptor forms a complex with the $\gamma$-aminobutyric acid (GABA) receptor and the $Cl^-$ ionophore, and BZPs exhibit pharmacological activity through the GABA response. That is, the compounds which act on the BZP receptors can be classified with different efficacy on a continuum from full agonists to inverse agonists, according to their modulatory effects on GABAergic transmission. Furthermore, they are classified into full agonists, partial agonists, antagonists, partial inverse agonists and inverse agonists in consideration of their behavioral pharmacological properties.

Recently, research works have emphasized the search for anxiolytic agents which selectively act on anxiety and have less side effects such as sedation, muscle-relaxation, potentiation of alcoholic effect or dependency liability associated with BZP agents. In the process of these investigations, many compounds have been found which possess high affinity for BZP receptors in spite of their different structures from BZP [JP-A H1(1989)-6278, JP-A H1(1989)-250383, JP-A H2(1990)-167285]. Among them, the compounds classified into BZP partial agonists have become important as a new type of anxiolytic agents since they are dissociated from side effects but exhibit selective pharmacological activity against anxiety.

[Disclosure of Invention]

The present inventors have conducted intensive investigations in order to develop anxiolytic agents having non-BZP structures and selective anxiolytic activity with high safety, and have found novel fused pyridazine compounds in which displayed anxioselective anxiolytic activity with less side effects because of not only high affinity for BZP receptors but also BZP partial agonistic property.

That is, the present invention provides:

1. a fused pyridazine compound of the formula:

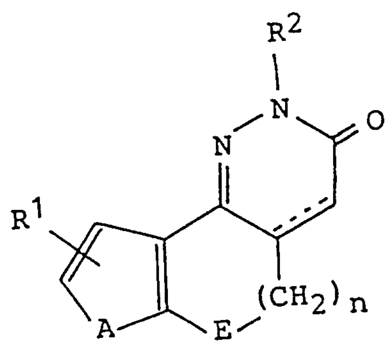

(I)

wherein:

$R^1$ is carboxyl, formyl, cyano, hydroxyiminomethyl, acyl, alkoxycarbonyl, aralkyloxycarbonyl, heteroarylalkyloxycarbonyl, aryloxycarbonyl, heteroaryloxycarbonyl, alkoxyalkyl, arylalkoxyalkyl, aryloxyalkyl, heteroaryloxyalkyl, acyloxyalkyl, hydroxyalkyl, acyloxyalkanoyl, alkoxyalkanoyl, hydroxyalkanoyl, aryloxyalkanoyl, haloalkanoyl, aralkyloxyalkanoyl, heteroarylalkyloxyalkanoyl or heteroaryloxyalkanoyl;

$R^2$ is hydrogen, alkyl having 1 to 8 carbon atoms, hydroxyalkyl, alkanoyloxyalkyl, aryl, aralkyl, heteroaryl, or aryl, aralkyl or heteroaryl having at least one substituent selected from the group consisting

of halogen, hydroxy, amino, nitro, cyano, alkyl having 1 to 4 carbon atoms, alkoxy having 1 to 4 carbon atoms, alkanoylamino having 2 to 5 carbon atoms, haloalkyl, acyloxy, alkoxycarbonyl having 2 to 5 carbon atoms and carboxyl on the aromatic ring;

A is sulphur atom or -CH=CH-;

E is $-CH_2-$ or $-S(O)_m-$ (m is 0, 1 or 2);

n is 1 or 2;

the bond represented by full line and dotted line is a single bond or a double bond;

providing that when A is sulphur atom, E is $-CH_2-$, and when A is -CH=CH-, E is $-S(O)_m-$ (m is 0, 1 or 2) and n is 2.

2. the fused pyridazine compound according to the above-mentioned item 1, wherein $R^2$ is aryl having at least one substituent selected from the group consisting of halogen, alkyl having 1 to 4 carbon atoms and alkoxy having 1 to 4 carbon atoms on the aromatic ring.

3. the fused pyridazine compound according to the above-mentioned item 1, wherein A is sulphur atom, and E is $-CH_2-$.

4. the fused pyridazine compound according to the above-mentioned item 1, wherein A is -CH=CH-, E is $-S(O)_m-$ (m is 0, 1 or 2) and n is 2.

5. the fused pyridazine compound according to the above-mentioned item 1, which is selected from the group consisting of:

8-acetyl-2-(4-chlorophenyl)-4,4a,5,6-tetrahydrothieno[2,3-h]cinnolin-3(2H)-one,
2-(4-chlorophenyl)-4,4a,5,6-tetrahydro-8-propionylthieno[2,3-h]cinnolin-3(2H)-one,
8-butyryl-2-(4-chlorophenyl)-4,4a,5,6-tetrahydrothieno[2,3-h]cinnolin-3(2H)-one,
8-benzoyl-2-(4-chlorophenyl)-4,4a,5,6-tetrahydrothieno[2,3-h]cinnolin-3(2H)-one,
8-acetyl-4,4a,5,6-tetrahydro-2-(4-methylphenyl)thieno[2,3-h]cinnolin-3(2H)-one,
9-acetyl-2-(4-chlorophenyl)-2,4,4a,5,6,7-hexahydro-3H-thieno[2',3':6,7]cyclohepta[1,2-c]pyridazin-3-one,
9-acetyl-2-(4-chlorophenyl)-2,5,6,7-tetrahydro-3H-thieno[2',3':6,7]cyclohepta[1,2-c]pyridazin-3-one,
2-(4-chlorophenyl)-9-formyl-2,5,6,7-tetrahydro-3H-thieno[2',3':6,7]cyclohepta[1,2-c]pyridazin-3-one,
8-(1-hydroxyethyl)-4,4a,5,6-tetrahydro-2-(4-methylphenyl)thieno[2,3-h]cinnolin-3(2H)-one,
8-acetyl-5,6-dihydro-2-(4-methylphenyl)thieno[2,3-h]cinnolin-3(2H)-one,
8-(1-hydroxyethyl)-5,6-dihydro-2-(4-methylphenyl)thieno[2,3-h]cinnolin-3(2H)-one,
8-chloroacetyl-4,4a,5,6-tetrahydro-2-(4-methylphenyl)thieno[2,3-h]cinnolin-3(2H)-one,
8-acetoxyacetyl-4,4a,5,6-tetrahydro-2-(4-methylphenyl)thieno[2,3-h]cinnolin-3(2H)-one,
8-(2-acetoxyethyl)-4,4a,5,6-tetrahydro-2-(4-methylphenyl)thieno[2,3-h]cinnolin-3(2H)-one,
4,4a,5,6-tetrahydro-8-(2-hydroxyethyl)-2-(4-methylphenyl)thieno[2,3-h]cinnolin-3(2H)-one,
10-acetyl-2-(4-chlorophenyl)-5,6-dihydro-[1]benzothiepino[5,4-c]pyridazin-3(2H)-one,
2-(4-chlorophenyl)-10-(1-hydroxyethyl)-5,6-dihydro-[1]benzothiepino[5,4-c]pyridazin-3(2H)-one,
10-acetyl-2-(4-chlorophenyl)-5,6-dihydro-[1]benzothiepino[5,4-c]pyridazin-3(2H)-one 7,7-dioxide,
2-(4-chlorophenyl)-10-(1-hydroxyethyl)-5,6-dihydro-[1]benzothiepino[5,4-c]pyridazin-3(2H)-one 7-oxide,
2-(4-chlorophenyl)-10-(1-formyloxyethyl)-5,6-dihydro-[1]benzothiepino[5,4-c]pyridazin-3(2H)-one 7-oxide,
2-(4-chlorophenyl)-5,6-dihydro-10-propionyl-[1]benzothiepino[5,4-c]pyridazin-3(2H)-one,
10-butyryl-2-(4-chlorophenyl)-5,6-dihydro-[1]benzothiepino[5,4-c]pyridazin-3(2H)-one,
2-(4-chlorophenyl)-10-(1-hydroxybutyl)-5,6-dihydro-[1]benzothiepino[5,4-c]pyridazin-3(2H)-one,
2-(4-chlorophenyl)-10-(1-hydroxyethyl)-5,6-dihydro-[1]benzothiepino[5,4-c]pyridazin-3(2H)-one 7,7-dioxide,
10-acetyl-2-(4-chlorophenyl)-5,6-dihydro-[1]benzothiepino[5,4-c]pyridazin-3(2H)-one 7-oxide,
10-chloroacetyl-2-(4-chlorophenyl)-5,6-dihydro-[1]benzothiepino[5,4-c]pyridazin-3(2H)-one,
methyl 2-(4-chlorophenyl)-2,3,5,6-tetrahydro-3-oxo-[1]benzothiepino[5,4-c]pyridazine-10-carboxylate, and
2-(4-chlorophenyl)-2,3,5,6-tetrahydro-3-oxo-[1]benzothiepino[5,4-c]pyridazine-10-carboxylic acid.

6. the fused pyridazine compound according to the above-mentioned item 1, wherein the compound of formula (I) is 9-acetyl-2-(4-chlorophenyl)-2,4,4a,5,6,7-hexahydro-3H-thieno[2',3':6,7]cyclohepta[1,2-c]-pyridazin-3-one.

7. a pharmaceutical composition comprising a therapeutically effective amount of the compound of the above-mentioned item 1 and pharmaceutically acceptable additives.

8. an anxiolytic agent containing a comound of the above-mentioned item 1 as an effective ingredient.

With regard to the above-mentioned formula (I), in the $R^1$, acyl means alkanoyl having 2 to 5 carbon atoms such as acetyl, propionyl, butyryl or isobutyryl; arylalkanoyl wherein the alkanoyl moiety has 2 to 5 carbon atoms such as phenylacetyl, 3-phenylpropionyl or 2-phenylpropionyl; arylcarbonyl such as benzoyl, 1-naphthoyl or 2-naphthoyl; heteroarylcarbonyl such as 2-, 3- or 4-nicotinoyl, 2- or 3- thenoyl or 2- or 3-furoyl; or heteroarylalkanoyl wherein the alkanoyl moiety has 2 to 5 carbon atoms such as pyridylacetyl, pyridylpropionyl, thienylacetyl, thienylpropionyl, furylacetyl or furylpropionyl. Alkoxycarbonyl wherein the alkoxy moiety has 1 to 4 carbon atoms means methoxycarbonyl, ethoxycarbonyl, propoxycarbonyl, isopropoxycarbonyl, butoxycarbonyl or isobutoxycarbonyl. Aralkyloxycarbonyl means benzyloxycarbonyl, 2-phenylethoxycarbonyl or 3-phenylpropxycarbonyl. Heteroarylalkyloxycarbonyl wherein the alkyl moiety has 1 to 4 carbon atoms means pyridylmethoxycarbonyl, pyridylethoxycarbonyl, thienylmethoxycarbonyl, thienylethoxycarbonyl, furylmethoxycarbonyl or furylethoxycarbonyl. Aryloxycarbonyl means phenoxycarbonyl or naphthyloxycarbonyl. Heteroaryloxycarbonyl means pyridyloxycarbonyl, thienyloxycarbonyl or furyloxycarbonyl. Alkoxyalkyl wherein the alkoxy and alkyl moieties have respectively 1 to 4 carbon atoms means methoxymethyl, methoxyethyl, methoxypropyl, methoxybutyl, ethoxymethyl, ethoxyethyl ethoxypropyl or ethoxybutyl. Arylalkoxyalkyl wherein the alkoxy and alkyl moieties have respectively 1 to 4 carbon atoms means benzyloxymethyl, 2-phenylethoxymethyl or 3-phenylpropoxymethyl. Aryloxyalkyl wherein the alkyl moiety has 1 to 4 carbon atoms means phenoxymethyl, phenoxyethyl, phenoxypropyl, phenoxybutyl, 1-naphthoxymethyl, 1-naphthoxyethyl, 1-naphthoxypropyl, 1-naphthoxybutyl, 2-naphthoxymethyl, 2-naphthoxyethyl, 2-naphthoxypropyl or 2-naphthoxybutyl. Heteroaryloxyalkyl wherein the alkyl moiety has 1 to 4 carbon atoms means pyridyloxymethyl, thienyloxymethyl or furyloxymethyl. Acyloxyalkyl means alkanoyloxyalkyl wherein the alkanoyl moiety has 1 to 5 carbon atoms and the alkyl moiety has 1 to 4 carbon atoms such as 1-formyloxyethyl, 1-formyloxypropyl, acetoxymethyl, propionyloxymethyl, 1- or 2-acetoxyethyl, 1- or 2-propionyloxyethyl, 1- or 3-acetoxypropyl or 1- or 3-propionyloxypropyl; aroyloxyalkyl wherein the alkyl moiety has 1 to 4 carbon atoms such as benzoyloxymethyl, benzoyloxyethyl, benzoyloxypropyl or benzoyloxybutyl; heteroarylcarbonyloxyalkyl wherein the alkyl moiety has 1 to 4 carbon atoms such as nicotinoyloxymethyl, thenoyloxymethyl or furoyloxymethyl; arylalkanoyloxyalkyl wherein the alkanoyl moiety has 2 to 5 carbon atoms and the alkyl moiety has 1 to 4 carbon atoms such as phenylacetyloxymethyl, phenylpropionyloxymethyl or phenylacetyloxyethyl; or heteroarylalkanoyloxyalkyl wherein the alkanoyl moiety has 2 to 5 carbon atoms and the alkyl moiety has 1 to 4 carbon atoms such as pyridylacetyloxymethyl, pyridylpropionyloxymethyl, thienylacetyloxymethyl, thienylpropionyloxymethyl furylacetyloxymethyl or furylpropionyloxymethyl. Hydroxyalkyl wherein the alkyl moiety has 1 to 4 carbon atoms means hydroxymethyl, 1- or 2-hydroxyethyl, 1-, 2- or 3-hydroxypropyl or 1-, 2-, 3- or 4-hydroxybutyl. Acyloxyalkanoyl means alkanoyloxyalkanoyl wherein both alkanoyl moieties have 2 to 5 carbon atoms such as acetoxyacetyl, acetoxypropionyl or acetoxybutyryl; arylcarbonyloxyalkanoyl wherein the alkanoyl moiety has 2 to 5 carbon atoms such as benzoyloxyacetyl, benzoyloxypropionyl or benzoyloxybutyryl; arylalkanoyloxyalkanoyl wherein both alkanoyl moieties have 2 to 5 carbon atoms such as phenylacetyloxyacetyl or phenylacetyloxypropionyl; heteroarylcarbonyloxyalkanoyl wherein the alkanoyl moiety has 2 to 5 carbon atoms such as nicotinoyloxyacetyl, thenoyloxyacetyl or furoyloxyacetyl; or heteroarylalkanoyloxyalkanoyl wherein both alkanoyl moieties have 2 to 5 carbon atoms such as pyridylacetyloxyacetyl, thienylacetyloxyacetyl or furylacetyloxypropionyl. Alkoxyalkanoyl wherein the alkoxy moiety has 1 to 4 carbon atoms and the alkanoyl moiety has 2 to 5 carbon atoms means methoxyacetyl, ethoxyacetyl, propoxyacetyl, butoxyacetyl, methoxypropionyl, ethoxypropionyl, propoxypropionyl or butoxypropionyl. Hydroxyalkanoyl wherein the alkanoyl moiety has 2 to 5 carbon atoms means hydroxyacetyl, hydroxypropionyl or hydroxybutyryl. Aryloxyalkanoyl wherein the alkanoyl moiety has 2 to 5 carbon atoms means phenoxyacetyl, phenoxypropionyl or phenoxybutyryl. Haloalkanoyl wherein the alkanoyl moiety has 2 to 5 carbon atoms means bromoacetyl, chloroacetyl, bromopropionyl, chloropropionyl, bromobutyryl, chlorobutyryl or trifluoroacetyl. Aralkyloxyalkanoyl wherein the alkanoyl moiety has 2 to 5 carbon atoms means benzyloxyacetyl, benzyloxypropionyl or 2-phenylethoxyacetyl. Heteroarylalkyloxyalkanoyl wherein the alkyl moiety has 1 to 4 carbon atoms and the alkanoyl moiety has 2 to 5 carbon atoms means pyridylmethoxyacetyl, thienylmethoxyacetyl, furylmethoxyacetyl, pyridylethoxyacetyl, thienylethoxypropionyl or furylethoxyacetyl. Heteroaryloxyalkanoyl wherein the alkanoyl moiety has 2 to 5 carbon atoms means pyridyloxyacetyl, thienyloxyacetyl or furyloxyacetyl.

In the $R^2$, alkyl having 1 to 8 carbon atoms means straight or branched chain alkyl such as methyl, ethyl, propyl, isopropyl, butyl, sec-butyl, tert-butyl, pentyl, isopentyl, neopentyl, hexyl, heptyl, octyl or 2-ethylhexyl. Hydroxyalkyl wherein the alkyl moiety has 1 to 4 carbon atoms means hydroxymethyl, 1- or 2-hydroxyethyl, 1-, 2- or 3-hydroxypropyl or 1-, 2-, 3- or 4-hydroxybutyl. Alkanoyloxyalkyl wherein the alkanoyl moiety has 2 to 5 carbon atoms and the alkyl moiety has 1 to 4 carbon atoms means acetoxymethyl, acetoxyethyl, acetoxypropyl, acetoxybutyl, propionyloxymethyl, propionyloxyethyl, propionyloxypropyl or propionyloxybutyl. Aryl means phenyl or naphthyl. Aralkyl means alkyl having 1 to 4 carbon atoms substituted by aryl such as benzyl, phenylethyl, phenylpropyl, phenylbutyl, naphthylmethyl, naphthylethyl, naphthylpropyl or naphthylbutyl. Heteroaryl means pyridyl, thienyl, furyl, pyrazolyl, imidazolyl, pyrimidinyl, pyridazinyl or benzimidazolyl. And halogen means chloroine, bromine, fluorine or iodine. Alkyl having 1 to 4 carbon atoms means methyl, ethyl, propyl, isopropyl, butyl, isobutyl or tert-butyl. Alkoxy having 1 to 4 carbon atoms means methoxy, ethoxy, propoxy, isopropoxy, butoxy or tert-butoxy. Acyloxy means alkanoyloxy having 2 to 5 carbon atoms such as acetyloxy, propionyloxy, butyryloxy or isobutyryloxy; or benzoyloxy. Haloalkyl wherein the alkyl moiety has 1 to 4 carbon atoms means fluoromethyl, bromomethyl, chloromethyl, iodomethyl, difluoromethyl, trifluoromethyl, 2,2-difluoroethyl or 2,2,2-trifluoroethyl. Alkoxycarbonyl having 2 to 5 carbon atoms means methoxycarbonyl, ethoxycarbonyl, propoxycarbonyl isopropoxycarbonyl, butoxycarbonyl, isobutoxycarbonyl or tert-butoxycarbonyl. Alkanoylamino having 2 to 5 carbon atoms means acetylamino, propionylamino, butyrylamino or pivaloylamino.

The compound of formula (I) having a chiral atom can be prepared as a racemate or an optically active isomer, and the compound having at least two chiral atoms can be obtained as an individual diastereomer or a mixture thereof. The present invention embraces the mixture thereof and the individual isomers. Furthermore, the present invention embraces stereoisomers.

When the compound of formula (I) contains carboxyl group as a substituent, the present invention includes a metal salt thereof with sodium, potassium or calcium, an amine salt thereof with methylamine, diethylamine or triethylamine, or an amino acid salt thereof with lysine, ornithine or arginine.

The compounds of the present invention can be prepared by the following methods.

Method (1)

A compound of the formula

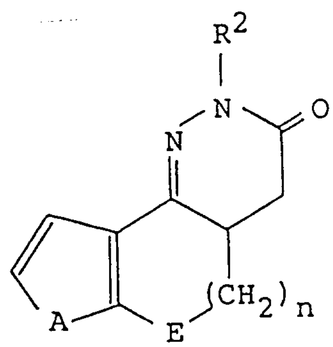

(1)

wherein each symbol is as defined above, is reacted with a carboxylic acid of the formula

$R^3COOH$ (2)

wherein $R^3$ is alkyl, haloalkyl, alkoxyalkyl, aryloxyalkyl, aryl, aralkyl, heteroaryl or heteroarylalkyl, or a reactive derivative thereof (e.g. acid halide, acid anhydride, mixed acid anhydride or ester) to produce a compound of the formula

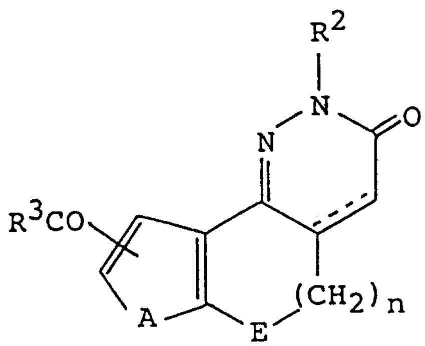

(3)

wherein each symbol is as defined above.

In the case where a free carboxylic acid compound of the formula (2) is employed, the reaction is conducted in the presence of a dehydrating agent (e.g. polyphosphoric acid) at room temperature to 150 °C.

In the case where an acid halide (e.g. acid chloride, acid bromide or acid iodide) as the reactive derivative of the formula (2) is employed, the reaction is conducted in the presence of Lewis acid (e.g. aluminum chloride, tin chloride or iron chloride) in a suitable inert solvent (e.g. benzene, toluene, chloroform, methylene chloride or dichloroethane) at -10 °C to 100 °C for 5 minutes to 20 hours.

Method (2)

The compound obtained in the Method (1) is subjected to a chemical reduction with a reducing agent such as sodium borohydride, lithium aluminum hydride or triethylsilane in a suitable inert solvent (e.g. methanol, ethanol, propanol, butanol or acetic acid), or a catalytic reduction in the presence of palladium, rhodium or platinum at -10 °C to 150 °C for 5 minutes to 20 hours to produce the compound of formula (I) wherein $R^1$ is 1-hydroxyalkyl, lower alkoxyalkyl, aryloxyalkyl and so on. The resulting compound is represented by the formula

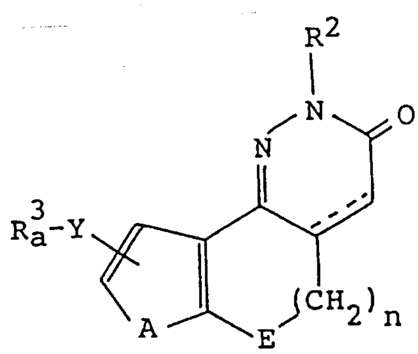

(4)

wherein $R^3a$ is alkyl, alkoxyalkyl, aryloxyalkyl or heteroaryloxyalkyl, and when $R^3a$ is alkyl, Y is -CH(OH)-, when $R^3a$ is alkoxyalkyl, aryloxyalkyl or heteroaryloxyalkyl, Y is $-CH_2-$, and other symbols are as defined above.

Method (3)

The compound obtained in the Method (2) represented by the formula

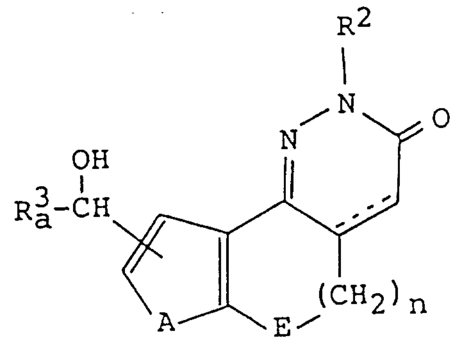

(4a)

wherein $R^3a$ is alkyl and other symbols are as defined above, is reacted with a carboxylic acid of the formula

$R^4COOH$ (5)

wherein $R^4$ is hydrogen, alkyl, aryl, aralkyl or heteroaryl, or an acid anhydride thereof, to produce a compound of the formula

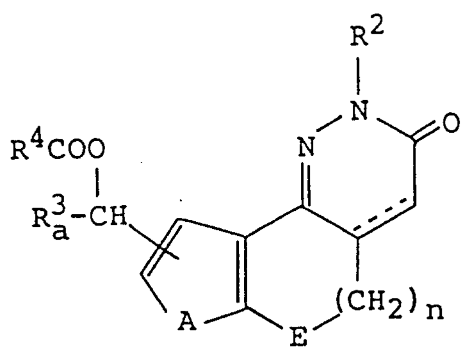

(6)

wherein each symbol is as defined above.

The reaction is carried out in a suitable inert solvent (e.g. benzene or toluene) or without solvent in the presence or absence of a base such as triethylamine at room temperature to 150 ° C.

Method (4)

The compound obtained in the Method (1) represented by the formula

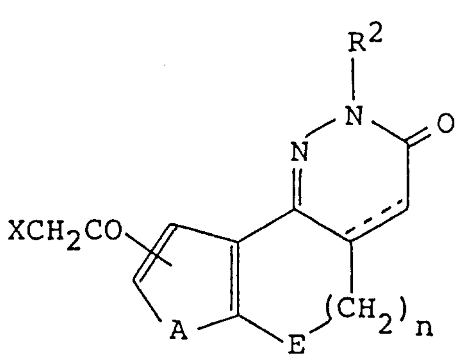

(3a)

wherein X is halogen and other symbols are as defined above, is reacted with pyridine, and the resulting pyridinium compound of formula

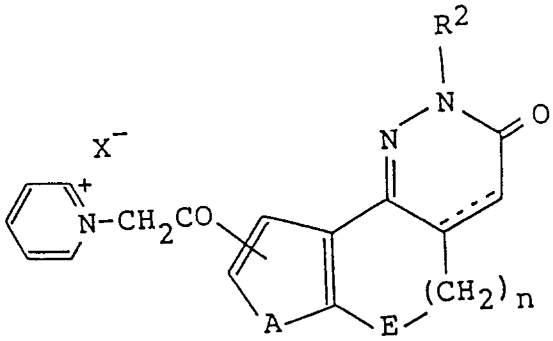

(7)

wherein each symbol is as defined above, is hydrolyzed to produce a compound of the formula

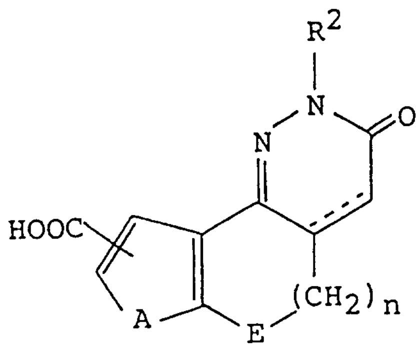

(8)

wherein each symbol is as defined above.

The reaction is carried out in a suitable inert solvent (e.g. water, methanol, ethanol or butanol) in the presence of an alkali (e.g. sodium hydroxide, potassium hydroxide or barium hydroxide) at room temperature to the refluxing temperature of the solvent employed for 1 to 10 hours.

Method (5)

The compound of formula (8) obtained in the Method (4) is kept in an alcohol (e.g. methanol, ethanol or butanol) in the presence of a suitable acid catalyst (e.g. sulfuric acid, hydrochloric acid, phosphoric acid or p-toluene-sulfonic acid) at room temperature to the refluxing temperature of the solvent employed for 1 to 24 hours, or an acid halide of the compound of formula (8) and an alcohol such as phenol, naphthol, benzyl alcohol or phenethyl alcohol are kept in a suitable solvent (e.g. benzene, toluene or methylene chloride) in the presence of an acid scavenger (e.g. triethylamine, pyridine, potassium carbonate or sodium hydrogen carbonate) at room temperature to the refluxing temperature of the solvent employed for 1 to 24 hours to produce a compound of the formula

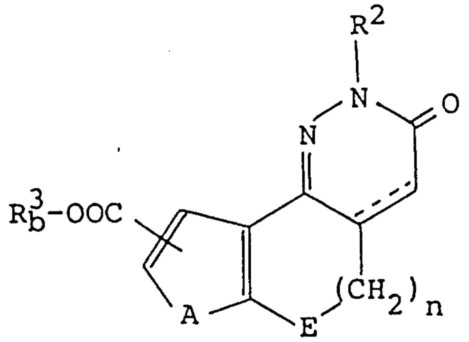

(9)

wherein $R^3b$ is alkyl, aryl, aralkyl, heteroaryl or heteroarylalkyl and other symbols are as defined above.

Method (6)

The haloalkanoyl compound obtained in the Method (1) represented by the formula

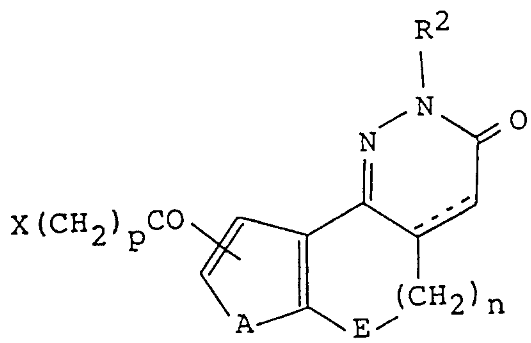

(3b)

wherein p is 1 to 3 and other symbols are as defined above, is reacted with a metal salt (e.g sodium salt, potassium salt or lithium salt) of a carboxylic acid compound of the formula

$R^5COOH$ (10)

wherein $R^5$ is alkyl, aryl, aralkyl, heteroaryl or heteroarylalkyl.

The reaction is carried out in a suitable inert solvent (e.g. acetic acid, chloroform, methylene chloride, benzene, toluene or N,N-dimethylformamide) at room temperature to 150 °C for 1 to 20 hours to produce a compound of the formula

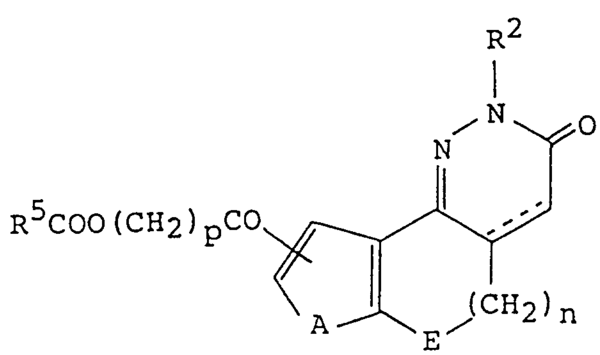

(11)

wherein each symbol is as defined above.

Further, the compound of formula (11) is reduced in a similar manner as in the Method (2) to produce a compound of the formula

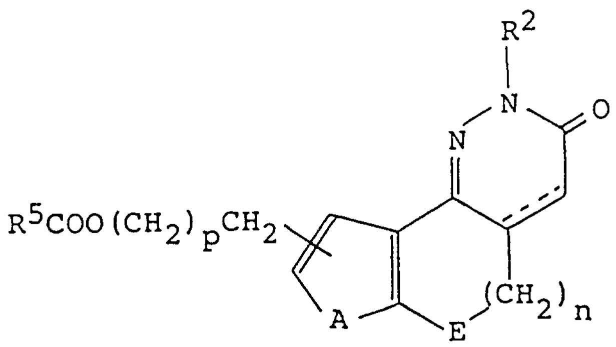
(12)

wherein each symbol is as defined above.

Method (7)

The compound of formula (3b) is reacted with a metal salt (e.g. sodium salt, potassium salt or lithium salt) of an alcoholic compound represented by the formula

$R^5OH$ (13)

wherein $R^5$ is as defined above, in a suitable inert solvent (e.g. methanol, ethanol, tetrahydrofuran, benzene, toluene or N,N-dimethylformamide) at room temperature to 150 °C for 1 to 20 hours to produce a compound of the formula

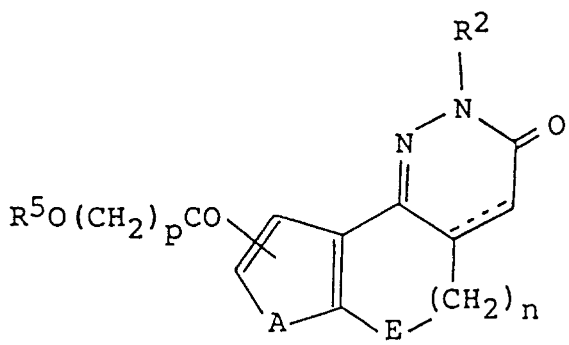
(14)

wherein each symbol is as defined above.

Method (8)

A compound of the formula

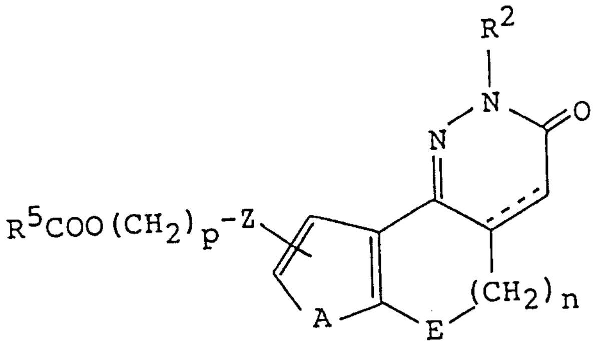

(15)

wherein Z is $CH_2$ or CO and other symbols are as defined above, is reacted with an aqueous solution of an acid (e.g. hydrochloric acid, sulfuric acid, phosphoric acid or nitric acid) or an alkali (e.g. sodium hydroxide, potassium hydroxide, lithium hydroxide, barium hydroxide or potassium carbonate) in a suitable inert solvent (e.g. acetic acid, methanol, ethanol, butanol or water) at -10 °C to 150 °C for 1 to 20 hours to produce a compound of the formula

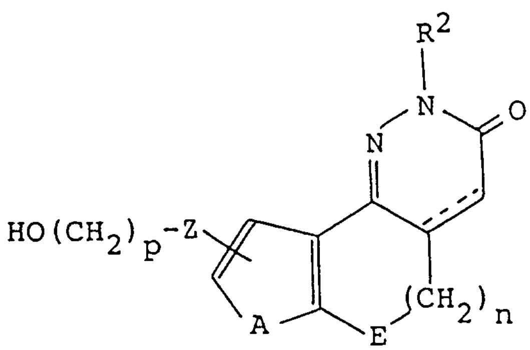

(16)

wherein each symbol is as defined above.

Method (9)

The compound of formula (16) is reacted with a compound of the formula

$R^6X$ (17)

wherein $R^6$ is alkyl, aryl or aralkyl and X is as defined above, in a suitable inert solvent (e.g. methanol, ethanol, propanol, butanol, N,N-dimethylformamide, tetrahydrofuran, benzene or toluene) in the presence of an acid scavenger (e.g. sodium hydride, sodium amide, sodium methoxide, sodium ethoxide, potassium hydroxide or sodium hydroxide) at room temperature to 150 °C for 1 to 20 hours to produce a compound of the formula

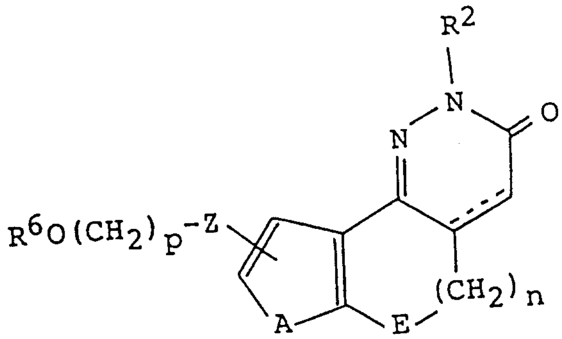

(18)

wherein each symbol is as defined above.

Method (10)

The pyridinium compound of formula (7) obtained in the Method (4) is reacted with a metal salt (e.g. sodium salt, potassium salt or lithium salt) of an alcoholic compound represented by the formula

$R^7OH$ (19)

wherein $R^7$ is alkyl, aryl, aralkyl or heteroaryl, to produce a compound of the formula

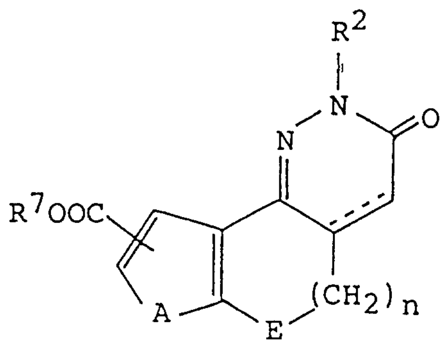

(20)

wherein each symbol is as defined above.

The reaction is carried out in a suitable inert solvent (e.g methanol, ethanol, propanol, benzene, toluene, tetrahydrofuran or N,N-dimethylformamide) at room temperature to the refluxing temperature of the solvent employed for 1 to 10 hours.

Method (11)

The compound of formula (I) wherein the bond represented by full line and dotted line is a double bond can also be prepared by adding bromine dropwise in an amount of 1 to 1.5 times mol to the corresponding compound of the formula (I) wherein the bond represented by full line and dotted line is a single bond in an acetic acid as the solvent at 20 °C to 60 °C [Journal of Medicinal Chemistry, vol. 14, 262 (1971)] or by reacting the compound of formula (I) wherein the bond represented by full line and dotted line is a single bond with sodium-m-nitrobenzenesulfonate (Bachmann method, the specification of United Kingdom Patent No. 1168291).

Method (12)

The compound of formula (1) is subjected to the Vilsmeier-Haack reaction to produce a compound of the formula

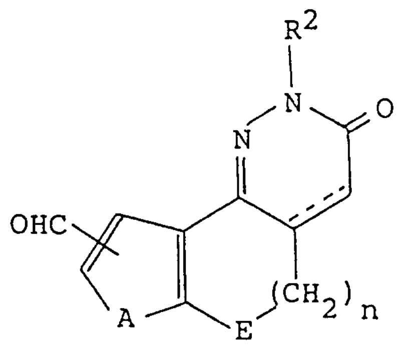

(21)

wherein each symbol is as defined above.

The reaction is carried out by reacting the compound of formula (I) with a formylation agent (e.g. N,N-dimethylformamide or N-methylformanilide) in the presence of phosphorus oxychloride at 0 ° C to 100 ° C for 10 minutes to 10 hours.

Method (13)

The compound of formula (21) is reduced by a reducing agent (e.g. sodium borohydride, sodium cyanoborohydride or lithium aluminum hydride) in an inert solvent (e.g. methanol, ethanol, propanol, butanol or tetrahydrofuran) at -10 ° C to 50 ° C for 5 minutes to 3 hours to produce a compound of the formula

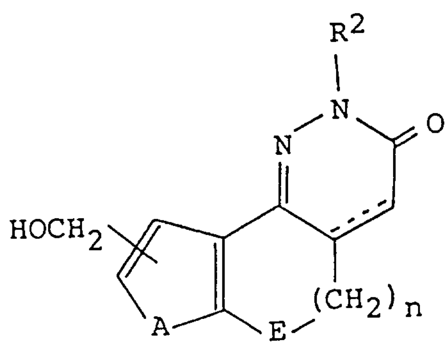

(22)

wherein each symbol is as defined above.

Method (14)

The compound of formula (21) is reacted with hydroxylamine hydrochloride in the presence of a suitable acid scavenger (e.g. sodium hydrogen carbonate, potassium carbonate, sodium hydroxide, potassium hydroxide or triethylamine) to produce a compound of the formula

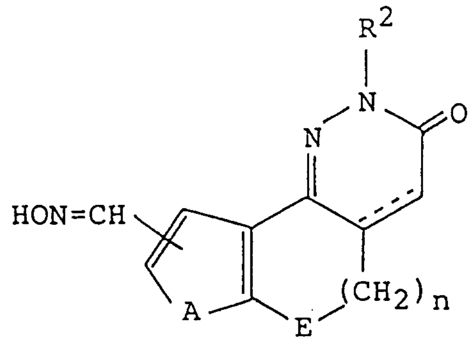

(23)

wherein each symbol is as defined above.

The reaction is carried out in a suitable inert solvent (e.g. ethanol, methanol, acetone, toluene or benzene) at room temperature to the boiling point of the solvent employed for 30 minutes to 20 hours.

Method (15)

The compound of formula (23) is reacted with a dehydrating agent such as acetic anhydride or trifluoroacetic anhydride to produce a compound of the formula

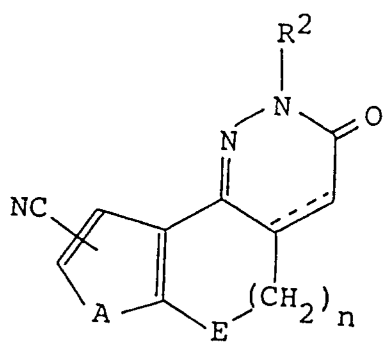

(24)

wherein each symbol is as defined above.

The reaction is carried out in a suitable inert solvent (e.g. tetrahydrofuran, methylene chloride, chloroform or toluene) in the presence or absence of a suiable acid scavenger such as triethylamine at -10 °C to the near boiling point of the solvent employed for 10 minutes to 24 hours.

Method (16)

The compound of formula (24) is reacted with an alcohol of the formula

$R^8OH$ (25)

wherein $R^8$ is lower alkyl, in the presence of a suitable acid (e.g. hydrochloric acid, sulfuric acid or phosphoric acid) to produce a compound of the formula

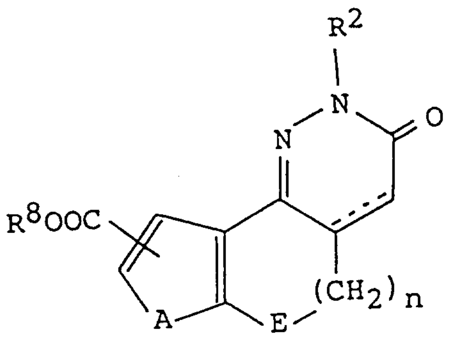

(26)

wherein each symbol is as defined above.

The reaction is carried out in a suitable inert solvent (e.g. toluene or benzene) or without solvent at room temperature to 100 ° C for 1 to 24 hours.

Method (17)

The compound of formula (I) wherein E is $-S(O)_m-$ and m is 1 or 2, can be prepared by subjecting a compound of the formula (I) wherein m is 0 to an oxidation reaction. The reaction is carried out by keeping the reaction system at -10 ° C to 100 ° C for 5 minutes to 20 hours in the presence of an oxidizing agent (e.g. hydrogen peroxide, peracetic acid, perbenzoic acid, m-chloroperbenzoic acid or sodium hypobromite) in a suitable inert solvent (e.g. acetic acid, formic acid, chloroform or methylene chloride).

The thus obtained compounds of the present invention can be isolated and purified by a conventional method such as recrystallization or column chromatography.

When the obtained compound is a racemate, it can be separated into desired optically active isomers by means of, for example, fractional recrystallization of a salt with an optically active acid or column chromatography filled with an optically active carrier. The individual diastereomers can be separated by the method such as fractional crystallization or chromatography. Such compounds can also be obtained by using an optically active starting material. Furthermore, the stereoisomers can be isolated by, for example, recrystallization or column chromatography.

Among the compounds of formula (1) which are starting materials, the compound wherein the bond represented by full line and dotted line is a single bond, that is, the compound of formula

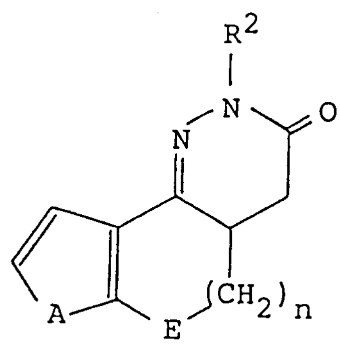

(1a)

wherein each symbol is as defined above, can be prepared by reacting a compound of the formula

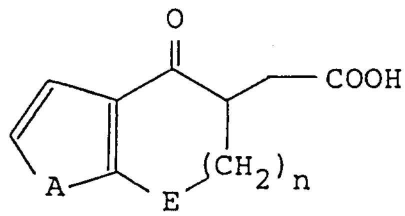

(27)

wherein each symbol is as defined above, with a hydrazine derivative of the formula

$R^2$-$NHNH_2$ (28)

wherein $R^2$ is as defined above, or an acid addition salt thereof, and then subjecting the thus obtained compound of formula

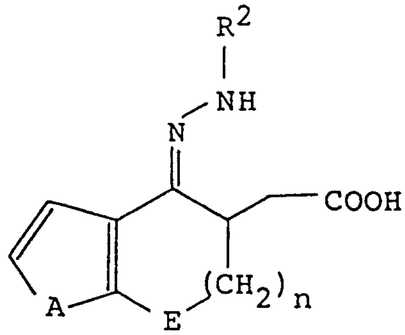

(29)

wherein each symbol is as defined above, to a ring closure reaction.

The reaction is carried out by refluxing the mixture under heating for 5 to 20 hours in a suitable inert solvent (e.g. alcohol such as methanol, ethanol or propanol, or benzene, toluene) to produce the compounds of formula (1a) and (29).

In the case where the hydrazine derivative of formula (28) is an acid addition salt thereof, the reaction is carried out in the presence of an acid scavenger (e.g. sodium acetate, potassium acetate, sodium hydrogen carbonate, sodium carbonate, potassium carbonate, pyridine or triethylamine).

In the case where the compound of formula (29) is obtained, the compound of formula (1a) can be prepared by refluxing the mixture under heating for 5 to 10 hours in acetic acid.

Further, the compound of formula (1) wherein the bond represented by full line and dotted line is a double bond, that is, the compound of formula

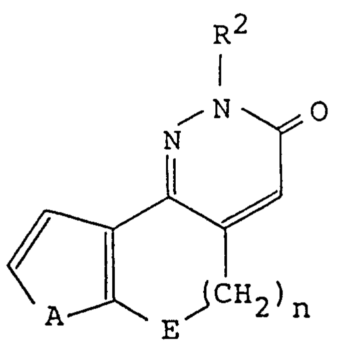

(1b)

wherein each symbol is as defined above, can be prepared by oxidizing the compound of formula (1a).

The reaction is carried out in a suitable acidic solvent (e.g. acetic acid, trifluoroacetic acid or methanesulfonic acid) in the presence of a suitable sulfoxide (e.g. dimethyl sulfoxide, diphenyl sulfoxide or

methyl phenyl sulfoxide) with or without a suitable acid (e.g. hydrogen halides such as hydrogen bromide) at 0 ° C to near the boiling point of the solvent employed to produce the compound of formula (1b).

Experiment 1: Displacement ability for benzodiazepine receptor

The test of specific binding to benzodiazepine (BZP) receptors was carried out according to the method described in Life Science, vol. 20, 2101 (1977).

The crude synaptosome fraction isolated from the cerebral cortex of male Wistar rats aged 9-10 weeks, was suspended in 50 mM Trishydrochloric acid buffer (pH 7.4) containing 120 mM sodium chloride and 5 mM potassium chloride. The suspension was used for the following experiment.

The test compound in several different concentrations and tritiated diazepam (in final concentration of 2 nM) were added to the synaptosomal suspension, and the mixture was incubated at 0 ° C for 20 minutes. The suspension was filtered with Whatman GF/B (Trademark) glassfiber filter. After the filter was washed with the above-mentioned buffer, the radio activity left on the filter was measured with a liquid scintillation counter.

The specific binding was determined by subtracting the binding in the presence of $10^{-6}$ M unlabelled diazepam from total binding.

The affinity for benzodiazepine receptors of the compound of the present invention is evaluated from its displacement ability for tritiated diazepam at its binding site, which is represented by Ki value (nM). The results were shown in Table 1.

Experiment 2: Effects on GABA-induced chloride current (concentration-clamp method)

The test was carried out according to the method of Akaike et al. reported in Journal of Physiology (London), 379, 171 (1986) using sensory neurons isolated from dosal root ganglion of American bullfrogs. Neurons were voltage-clamped at a holding membrane potential of -50 mV with a single-electrode voltage-clamp system. Test compounds were applied by using a concentration-clamp technique. When the peak $Cl^-$ current ($I_{Cl}$) elicited by 3 x $10^{-6}$ M GABA alone was presented as 1, the augumentive $I_{Cl}$ of the test compounds in the presence of 3 x $10^{-6}$ M GABA was measured. The results were presented as relative $I_{Cl}$ values in Table 1.

Table 1

| Test compound (Example No.) | BZP receptor binding Ki (nM) | Relative $I_{Cl}$ 1) |
|---|---|---|
| 18 | 4.2 | 1.38 |

1) $I_{Cl}$ = above ca. 2 : full agonist
$I_{Cl}$ = ca. 1.3 to ca. 1.9 : partial agonist
[Br. J. Pharmacol., vol. 98, 735-740 (1989)]

Experiment 3: Anti-conflict action

The test is carried out according to the method of Vogel et al. [Psychopharmacology, 21, 1 (1971)]. Groups of 10 to 14 male Wistar rats weighing 150 - 200 g are used. They are deprived of water for 72 hours before the test. The experimental apparatus is composed of a light compartment and a dark compartment equipped with a nozzle for water supply, where the rats are allowed to ambulate between the two compartments. One hour after the oral administration of the test compound, the rat is placed into the test apparatus where an electric shock is given once every 20th lick through the nozzle and grid floor. After the rat receive the first electric shock, the number of shocks are recorded during the subsequent 3 minutes test period. The minimum effective dose (MED) is defined as the lowest dose producing a statistically significant difference from methyl cellulose-treated group by t-test.

Experiment 4: Muscle-relaxant effect

Groups of 10 male ddY mice are used. The mice are gently placed on the rod (2.8 cm in diameter rotating at 11 r.p.m.) one hour after the oral administration of the test compound. The $ED_{50}$ value is calculated by the probit method as the dose which cause 50% of the animals to drop off the rotarod within 1 minute.

Experiment 5: Potentiation of narcotic effect

Groups of 7 male mice are administered orally with test compound and one hour later administered intraperitoneally with subnarcotic dose of hexobarbital at 40 mg/kg. The loss of righting reflex is determined at 15 and 30 minutes after hexobarbital treatment. The $ED_{50}$ (mg/kg) is calculated by the probit method as the dose which cause a loss of righting reflex for more than 3 seconds in 50% of the animals.

Experiment 6: Acute toxicity

Groups of 5 male ddY mice were used. The mice were administered with 300 mg/kg of the compound of Example 16 intraperitoneally, but all mice survived for 5 days after the administration. Similarly, the mice were orally administered with 1000 mg/kg of the compound, but they survived for 5 days after the administration.

As apparent from the various pharmacological studies including the above experiments, the compounds of formula (I) of the present invention possessed high affinity for BZP receptors, and exhibited antagonistic action against chemical convulsion, such as bicuculline.

Especially, since the compounds of the present invention had high affinity for type I receptor of BZP receptors and further showed the property as a BZP partial agonist in the electrophysiological experiment, they are fully dissociated from the side effects such as muscle-relaxation, sedation, interaction with alcohol/barbiturates etc. and are useful as an anxioselective anxiolytic agent. Furthermore, it is recognized that the compounds of the present invention show potent anxiolytic activity in in vivo experiments of the various animal models of anxiety such as water-lick method by Vogel et al, elevated plus maze method [Costall et al. (Br. J. Pharmacol., 96, 312 (1989)); Meert et al. (Drug. Develop. Res., 18, 119 (1989)); Singh et al. (Br. J. Pharmacol., 104, 239 (1991))], social interaction method [File et al. (J. Neurosci. Methods, 2, 219 (1980))] or light and dark compartments method [Jones et al. (Br. J. Pharmacol., 93, 985 (1988))] and that they are largely separated from the side effects (muscle-relaxation, potentiation of narcosism). Consequently, the compounds of the present invention can be used as anxiolytic agent with high safety.

When the compounds of formula (I) of the present invention are used as pharmaceuticals, a therapeutically effective amount of the compounds and adequate pharmaceutically acceptable additives such as excipient, carrier or diluent are mixed to be formulated into a form such as tablets, capsules, granules, syrups, injectable solutions, suppositories or dispersible powders and are administered in the form mentioned above. The dosage may generally range about 5 to about 500 mg per day for an adult in a single dose or divided doses in the case of oral administration.

The present invention will be explained in more detail by the following examples, but these examples are not to be construed as limiting the present invention.

Example 1

To a mixture of 102 g of 2-(4-chlorophenyl)-5,6-dihydro-[1]benzothiepino[5,4-c]pyridazin-3(2H)-one and 102 g of acetic anhydride in 1000 ml of dichloroethane was added 306 g of aluminum chloride under ice-cooling. The mixture was stirred at room temperature for 10 minutes and further stirred at 50 to 60 °C for 6 hours, and then poured into ice-cold water. The organic layer was collected by a separatory funnel and dried over magnesium sulfate. The solvent was concentrated in vacuo and the residue was recrystallized from a mixed solvent of chloroform and ethanol to give 64 g of 10-acetyl-2-(4-chlorophenyl)-5,6-dihydro-[1]-benzothiepino[5,4-c]pyridazin-3(2H)-one 1/3hydrate as a white crystal, melting at 178-179 °C.

Example 2

To a mixture of 64 g of 10-acetyl-2-(4-chlorophenyl)-5,6-dihyrdo-[1]benzothiepino[5,4-c]pyridazin-3(2H)-one 1/3hydrate in 500 ml of methanol and 500 ml of chloroform was added 6.4 g of sodium borohydride with stirring under ice-cooling. The mixture was stirred at room temperature for an hour and concentrated in

vacuo. To the residue was added water and the solution was extracted twice with chloroform. The chloroform layer was washed with water, dried over magnesium sulfate and concentrated. The residue was recrystallized from ethanol to give 59 g of 2-(4-chlorophenyl)-10-(1-hydroxyethyl)-5,6-dihydro-[1]-benzothiepino[5,4-c]pyridazin-3(2H)-one 1/2hydrate as a white crystal, melting at 122-124 °C.

Example 3

To a solution of 2 g of 10-acetyl-2-(4-chlorophenyl)-5,6-dihydro-[1]benzothiepino[5,4-c]pyridazin-3(2H)-one 1/3hydrate in 20 ml of formic acid was added 3 ml of 30% hydrogen peroxide solution. After the mixture was stirred at room temperature for 5 hours, the mixture was poured into water and the precipitate was collected by filtration. The precipitate was recrystallized from dimethylformamide to give 1.9 g of 10-acetyl-2-(4-chlorophenyl)-5,6-dihydro-[1]benzothiepino[5,4-c]pyridazin-3(2H)-one 7,7-dioxide as a white crystal, melting at 274-275 °C.

Example 4

To a solution of 2.66 g of 2-(4-chlorophenyl)-10-(1-hydroxyethyl)-5,6-dihydro-[1]benzothiepino[5,4-c]-pyridazin-3(2H)-one 1/2hydrate in 40 ml of acetic acid was added 1.57 ml of 30% hydrogen peroxide solution. After the mixture was stirred at room temperature for 4 hours, the mixture was poured into water and the precipitate was collected by filtration. The precipitate was recrystallized from a mixed solvent of ethanol and isopropyl ether to give 2.17 g of 2-(4-chlorophenyl)-10-(1-hydroxyethyl)-5,6-dihydro-[1]-benzothiepino[5,4-c]pyridazin-3(2H)-one 7-oxide as a white crystal, melting at 194-196 °C.

Example 5

To a solution of 2.5 g of 2-(4-chlorophenyl)-10-(1-hydroxyethyl)-5,6-dihydro-[1]benzothiepino[5,4-c]-pyridazin-3(2H)-one 1/2hydrate in 20 ml of formic acid was added 0.7 ml of 30% hydrogen peroxide solution. After the mixture was stirred at room temperature for an hour, the mixture was poured into water and extracted with chloroform. The organic layer was washed with acidic sodium hydrogensulfite solution, sodium hydrogen carbonate solution and then water. The chlorofom layer was dried over magnesium sulfate and concentrated. The residue was chromatographed on a silica gel column. The resulting solid was recrystallized from ethanol to give 1.1 g of 2-(4-chlorophenyl)-10-(1-formyloxyethyl)-5,6-dihydro-[1]-benzothiepino[5,4-c]pyridazin-3(2H)-one 7-oxide as a white crystal, melting at 195-196 °C.

Example 6

The reaction and procedure were conducted by in a similar manner as in Example 1 using 2-(4-chlorophenyl)-5,6-dihydro-[1]benzothiepino[5,4-c]pyridazin-3(2H)-one and propionyl chloride to give 2-(4-chlorophenyl)-5,6-dihydro-10-propionyl-[1]benzothiepino[5,4-c]pyridazin-3(2H)-one, melting at 197-198 °C.

Example 7

The reaction and procedure were conducted by in a similar manner as in Example 1 using 2-(4-chlorophenyl)-5,6-dihydro-[1]benzothiepino[5,4-c]pyridazin-3(2H)-one and butyryl chloride to give 10-butyryl-2-(4-chlorophenyl)-5,6-dihydro-[1]benzothiepino[5,4-c]pyridazin-3(2H)-one, melting at 179-180 °C.

Example 8

The reaction and procedure were conducted by in a similar manner as in Example 2 using 10-butyryl-2-(4-chlorophenyl)-5,6-dihydro-[1]benzothiepino[5,4-c]pyridazin-3(2H)-one to give 2-(4-chlorophenyl)-10-(1-hydroxybutyl)-5,6-dihydro-[1]benzothiepino[5,4-c]pyridazin-3(2H)-one, melting at 169-170 °C.

Example 9

The reaction and procedure were conducted by in a similar manner as in Example 3 using 2-(4-chlorophenyl)-10-(1-hydroxyethyl)-5,6-dihydro-[1]benzothiepino[5,4-c]pyridazin-3(2H)-one to give 2-(4-chlorophenyl)-10-(1-hydroxyethyl)-5,6-dihydro-[1]benzothiepino(5,4-c]pyridazin-3(2H)-one 7,7-dioxide, melting at 250-251 °C.

Example 10

The reaction and procedure were conducted by in a similar manner as in Example 4 using 10-acetyl-2-(4-chlorophenyl)-5,6-dihydro-[1]benzothiepino[5,4-c]pyridazin-3(2H)-one to give 10-acetyl-2-(4-chlorophenyl)-5,6-dihydro-[1]benzothiepino[5,4-c]pyridazin-3(2H)-one 7-oxide 1/3hydrate, melting at 231-232 °C with decomposition.

Example 11

To a mixture of 34 g of 2-(4-chlorophenyl)-5,6-dihydro-[1]benzothiepino[5,4-c]pyridazin-3(2H)-one and 34 g of chloroacetyl chloride in 250 ml of dichloroethane was added 53.2 g of anhydrous aluminum chloride keeping the temperature below 10 °C and the mixture was stirred at the same temperature for 30 minutes. After the mixture was stirred at 50±5 °C for 5 hours, the mixture was poured into ice-cold water and then allowed to stand overnight. The resulting solid was collected by filtration, washed with water and recrystallized from ethanol to give 24.5 g of 10-chloroacetyl-2-(4-chlorophenyl)-5,6-dihydro-[1]benzothiepino[5,4-c]-pyridazin-3(2H)-one, melting at 208-210 °C.

Example 12

A solution of 10.0 g of 10-chloroacetyl-2-(4-chlorophenyl)-5,6-dihydro-[1]benzothiepino[5,4-c]pyridazin-3-(2H)-one in 150 ml of pyridine was kept at 80 °C for 5 hours and then concentrated in vacuo. To the residue was added a small amount of ethanol. The precipitate was collected by filtration and recrystallized from ethanol to give 10.1 g of 1-[2-(4-chlorophenyl)-2,3,5,6-tetrahydro-3-oxo-[1]benzothiepino[5,4-c]pyridazin-10-ylcarbonylmethyl]pyridinium chloride, melting at 188-190 °C with decomposition. To a solution of 2.5 g of 1-[2-(4-chlorophenyl)-2,3,5,6-tetrahydro-3-oxo-[1]benzothiepino[5,4-c]pyridazin-10-ylcarbonylmethyl]pyridinium chloride in 100 ml of methanol was added 0.4 g of sodium methoxide keeping the temperature below 5 °C and the mixture was allowed to stand at the same temperature for 30 minutes. The mixture was concentrated in vacuo, a saturated ammonium chloride solution was added thereto and extracted with ethyl acetate. The extract was washed with water, dried over anhydrous magnesium sulfate and concentrated in vacuo. The resulting solid was recrystallized from ethanol to give 0.3 g of methyl 2-(4-chlorophenyl)-2,3,5,6-tetrahydro-3-oxo-[1]benzothiepino[5,4-c]pyridazine-10-carboxylate, melting at 177-178 °C with decomposition. On the other hand, the precipitate in the aqueous layer was collected by filtration and washed with diluted sodium hydroxide solution. The obtained filtrate was acidified with diluted hydrochloric acid. The resulting crystal was collected by filtration, washed with water and recrystallized from methanol to give 0.4 g of 2-(4-chlorophenyl)-2,3,5,6-tetrahydro-3-oxo-[1]benzothiepino[5,4-c]pyridazine-10-carboxylic acid, melting at a temperature above 280 °C.

Example 13

To a suspension of 5.7 g of aluminum chloride in 100 ml of methylene chloride was added 2.0 ml of acetyl chloride under ice-cooling and the mixture was stirred at room temperature for 10 minutes. 4.5 g of 2-(4-chlorophenyl)-4,4a,5,6-tetrahydrothieno[2,3-h]cinnolin-3(2H)-one was added under ice-cooling, and the mixture was refluxed for 2 hours. After cooling, the mixture was poured into ice-cold water and extracted with chloroform. The extract was washed with water, dried over anhydrous magnesium sulfate and the solvent was concentrated in vacuo. The resulting crystal was recrystallized from a mixed solvent of chloroform and ethanol to give 4.7 g of 8-acetyl-2-(4-chlorophenyl)-4,4a,5,6-tetrahydrothieno[2,3-h]cinnolin-3(2H)-one as a white crystal, melting at 155-157 °C.

Example 14

The reaction and procedure were conducted in a similar manner as in Example 13 using propionyl chloride instead of acetyl chloride to give 2-(4-chlorophenyl)-4,4a,5,6-tetrahydro-8-propionylthieno[2,3-h]-cinnolin-3(2H)-one as a white crystal, melting at 135-138 °C.

Example 15

The reaction and procedure were conducted in a similar manner as in Example 13 using butyryl chloride instead of acetyl chloride to give 8-butyryl-2-(4-chlorophenyl)-4,4a,5,6-tetrahydrothieno[2,3-h]-

cinnolin-3(2H)-one as a white crystal, melting at 157-159 ° C.

Example 16

The reaction and procedure were conducted in a similar manner as in Example 13 using benzoyl chloride instead of acetyl chloride to give 8-benzoyl-2-(4-chlorophenyl)-4,4a,5,6-tetrahydrothieno[2,3-h]-cinnolin-3(2H)-one as a white crystal, melting at 162-164 ° C.

Example 17

The reaction and procedure were conducted in a similar manner as in Example 13 using 4,4a,5,6-tetrahydro-2-(4-methylphenyl)thieno[2,3-h]cinnolin-3(2H)-one instead of 2-(4-chlorophenyl)-4,4a,5,6-tetrahydrothieno[2,3-h]cinnolin-3(2H)-one to give 8-acetyl-4,4a,5,6-tetrahydro-2-(4-methylphenyl)thieno[2,3-h]cinnolin-3(2H)-one as a white crystal, melting at 191-193 ° C.

Example 18

The reaction and procedure were conducted in a similar manner as in Example 13 using 2-(4-chlorophenyl)-2,4,4a,5,6,7-hexahydro-3H-thieno[2',3':6,7]cyclohepta[1,2-c]pyridazin-3-one instead of 2-(4-chlorophenyl)-4,4a,5,6-tetrahydrothieno[2,3-h]cinnolin-3(2H)-one to give 9-acetyl-2-(4-chlorophenyl)-2,4,4a,5,6,7-hexahydro-3H-thieno[2',3':6,7]cyclohepta[1,2-c]pyridazin-3-one as a white crystal, melting at 128-130 ° C.

Example 19

To a solution of 1.2 g of 9-acetyl-2-(4-chlorophenyl)-2,4,4a,5,6,7-hexahydro-3H-thieno[2',3':6,7]-cyclohepta[1,2-c]pyridazin-3-one in 15% hydrogen bromide-acetic acid solution was added 0.23 ml of dimethylsulfoxide at room temperature with stirring. After stirring at the same temperature for 15 minutes, the mixture was poured into 0.5% sodium hydrogensulfite solution and extracted with chloroform. The extract was washed with water, dried over anhydrous magnesium sulfate and concentrated. The residue was chromatographed on a silica gel column using chloroform as an eluent. The resulting solid was recrystallized from ethanol to give 0.4 g of 9-acetyl-2-(4-chlorophenyl)-2,5,6,7-tetrahydro-3H-thieno[2',3':6,7]-cyclohepta[1,2-c]pyridazin-3-one as a white crystal, melting at 164-166 ° C.

Example 20

A mixture of 0.45 g of N-methylformanilide in 0.33 ml of phosphrus oxychloride was stirred at room temperature for an hour and 0.5 g of 2-(4-chlorophenyl)-2,5,6,7-tetrahydro-3H-thieno[2',3':6,7]cyclohepta[1,2-c]pyridazin-3-one was added thereto. After stirring at room temperature for 2 hours, the mixture was poured into ice-cold water and extracted with ethyl acetate. The extract was washed with water, dried over anhydrous magnesium sulfate and concentrated in vacuo. The residue was chromatographed on a silica gel ($SiO_2$:40 g ) column using chloroform as an eluent. The resulting solid was recrystallized from a mixed solvent of chloroform and ethanol to give 0.25 g of 2-(4-chlorophenyl)-9-formyl-2,5,6,7-tetrahydro-3H-thieno-[2',3':6,7]-cyclohepta[1,2-c]pyridazin-3-one as a pale yellow powder, melting at 170-172 ° C.

Example 21

To a suspension of 1.3 g of 8-acetyl-4,4a,5,6-tetrahydro-2-(4-methylphenyl)thieno[2,3-h]cinnolin-3(2H)-one in methanol was added 300 mg of sodium borohydride under ice-cooling and the mixture was stirred for 3 hours. The mixture was concentrated in vacuo until about a half volume of the methanol was evaporated and water was added, and then extracted with chloroform. The extract was washed with brine, dried over anhydrous magnesium sulfate and concentrated in vacuo. The residue was chromatographed on a silica gel ($SiO_2$:30 g ) column using a mixed solvent of chloroform and methanol (98:2) as an eluent. The resulting solid was recrystallized from a mixed solvent of ethyl acetate and hexane to give 570 mg of 8-(1-hydroxyethyl)-4,4a,5,6-tetrahydro-2-(4-methylphenyl)thieno[2,3-h]cinnolin-3(2H)-one as a pale yellow powder, melting at 129-145 ° C.

Example 22

To a suspension of 6.4 g of aluminum chloride in methylene chloride was added 2.3 ml of acetyl chloride under ice-cooling and the mixture was stirred for 10 minutes. A solution of 4.7 g of 5,6-dihydro-2-(4-methylphenyl)thieno[2,3-h]cinnolin-3(2H)-one in methylene chloride was added thereto, the mixture was stirred at room temperature for 30 minutes and then refluxed for an hour. The mixture was poured into ice-cold water and extracted with chloroform. The extract was washed with water, dried over anhydrous magnesium sulfate and concentrated in vacuo. The residue was chromatographed on a silica gel ($SiO_2$:100 g) column using chloroform as an eluent. The resulting solid was recrystallized from a mixed solvent of chloroform and methanol to give 3.7 g of 8-acetyl-5,6-dihydro-2-(4-methylphenyl)thieno[2,3-h]cinnolin-3(2H)-one as a white powder, melting at 240-242 °C.

Example 23

To a suspension of 600 mg of 8-acetyl-5,6-dihydro-2-(4-methylphenyl)thieno[2,3-h]cinnolin-3(2H)-one in 100 ml of methanol was added 70 mg of sodium borohydride with stirring under ice-cooling. The mixture was stirred for 3 hours and poured into ice-cold water and then extracted with chloroform. The extract was washed with brine, dried over anhydrous magnesium sulfate and concentrated in vacuo. The residue was chromatographed on a silica gel ($SiO_2$:20 g) column using a mixed solvent of chloroform and methanol (98:2) as an eluent. The resulting solid was recrystallized from a mixed solvent of ethyl acetate and hexane to give 300 mg of 8-(1-hydroxyethyl)-5,6-dihydro-2-(4-methylphenyl)thieno[2,3-h]cinnolin-3(2H)-one as a pale yellow powder, melting at 146-148 °C.

Example 24

To a suspension of 20.3 g of aluminum chloride in methylene chloride was added 8.1 ml of chloroacetyl chloride under ice-cooling. A solution of 15 g of 4,4a,5,6-tetrahydro-2-(4-methylphenyl)thieno[2,3-h]cinnolin-3(2H)-one in methylene chloride was added thereto. The mixture was refluxed for 8 hours, and poured into ice-cold water and then extracted with chloroform. The extract was washed with water, dried over anhydrous magnesium sulfate and concentrated in vacuo. The residue was chromatographed on a silica gel ($SiO_2$:600 g) column using chloroform as an eluent. The starting material (7.2 g) was recovered from the first fraction and the desired compound was obtained from the second fraction. The crystal was recrystallized from a mixed solvent of chloroform and ethanol to give 6.5 g of 8-chloroacetyl-4,4a,5,6-tetrahydro-2-(4-methylphenyl)thieno[2,3-h]cinnolin-3(2H)-one as a pale yellow powder, melting at 169-171 °C.

Example 25

To a suspension of 5.8 g of 8-chloroacetyl-4,4a,5,6-tetrahydro-2-(4-methylphenyl)thieno[2,3-h]cinnolin-3-(2H)-one in 100 ml of acetic acid was added 12 g of potassium acetate and the mixture was stirred for 5 hours. The mixture was poured into ice-cold water and the precipitate was collected by filtration. The filtrate was extracted with ethyl acetate. The extract was washed with brine, dried over anhydrous magnesium sulfate and concentrated in vacuo. The residue and the precipitate were combined and chromatographed on a silica gel ($SiO_2$:200 g) column using chloroform as an eluent. The resulting crystals were recrystallized from a mixed solvent of chloroform and methanol to give 4.4 g of 8-acetoxyacetyl-4,4a,5,6-tetrahydro-2-(4-methylphenyl)thieno[2,3-h]cinnolin-3(2H)-one as a white powder, melting at 144-145 °C.

Example 26

To a solution of 3 g of 8-acetoxyacetyl-4,4a,5,6-tetrahydro-2-(4-methylphenyl)thieno[2,3-h]cinnolin-3-(2H)-one in 30 ml of trifluoroacetic acid was added 2.7 ml of triethylsilane and the mixture was stirred at room temperature for 24 hours. The mixture was poured into ice-cold water, neutrallized with potassium carbonate and then extracted with chloroform. The extract was washed with brine, dried over anhydrous magnesium sulfate and concentrated in vacuo. The residue was chromatographed on a silica gel ($SiO_2$:100 g) column using chloroform as an eluent. The resulting solid was recrystallized from a mixed solvent of ethyl acetate and hexane to give 2.0 g of 8-(2-acetoxyethyl)-4,4a,5,6-tetrahydro-2-(4-methylphenyl)thieno-[2,3-h]cinnolin-3(2H)-one as a white powder, melting at 73-75 °C.

Example 27

To a suspension of 1.5 g of 8-(2-acetoxyethyl)-4,4a,5,6-tetrahydro-2-(4-methylphenyl)thieno[2,3-h]-cinnolin-3(2H)-one in 50 ml of methanol was added 5 ml of aqueous solution of 0.7 g of potassium carbonate under ice-cooling. The mixture was stirred for an hour, and poured into water and then extracted with ethyl acetate. The extract was washed with brine, dried over anhydrous magnesium sulfate and concentrated in vacuo. The resulting solid was recrystallized from a mixed solvent of chloroform and ethanol to give 1.3 g of 4,4a,5,6-tetrahydro-8-(2-hydroxy-ethyl)-2-(4-methylphenyl)thieno[2,3-h]cinnolin-3-(2H)-one as a white powder, melting at 159-160 °C.

The compounds exemplified in the following tables can be prepared in a similar manner as the above examples.

Table 2

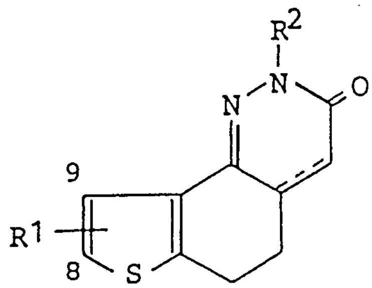

| No. | $R^1$ | $R^2$ | ==== bond |
|---|---|---|---|
| 28 | $8-COOH$ | 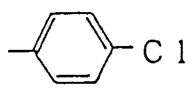 | single |
| 29 | $8-COCH_2Cl$ | 〃 | 〃 |
| 30 | $8-\underset{\mid}{C}HCH_3$ OH | 〃 | 〃 |
| 31 | $8-COCH_2OCOCH_3$ | 〃 | 〃 |
| 32 | $8-CH_2CH_2OCOCH_3$ | 〃 | 〃 |
| 33 | $8-COOCH_3$ | 〃 | 〃 |
| 34 | 〃 | 〃 | double |
| 35 | $8-COCF_3$ | 〃 | single |
| 36 | $8-COCH_3$ | 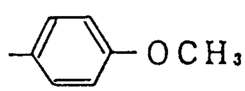 | 〃 |
| 37 | $8-\underset{\mid}{C}HCH_3$ OH | 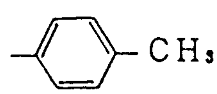 | 〃 |

Table 3

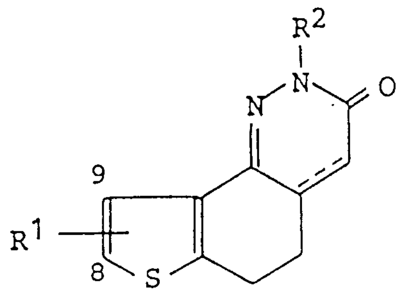

| No. | $R^1$ | $R^2$ | ==== bond |
|---|---|---|---|
| 38 | 8 − CO 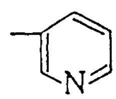 | 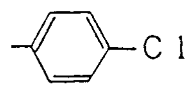 | single |
| 39 | $8-COCH_2$ 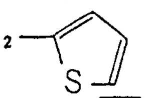 | 〃 | 〃 |
| 40 | $8-COOCH_2$ 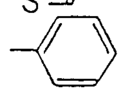 | 〃 | 〃 |
| 41 | 8 − COO 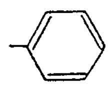 | 〃 | 〃 |
| 42 | $8-COOCH_2CH_2$ 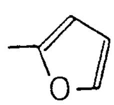 | 〃 | 〃 |
| 43 | $8-CH_2OCH_3$ | 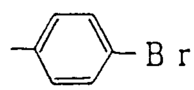 | 〃 |
| 44 | $8-CHCH_3$<br>OCHO | 〃 | 〃 |
| 45 | $8-CH_2OCO$ 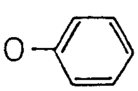 | 〃 | 〃 |
| 46 | $8-CH_2OCOCH_2$ 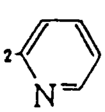 | 〃 | 〃 |
| 47 | $8-CH_2OH$ | 〃 | double |

Table 4

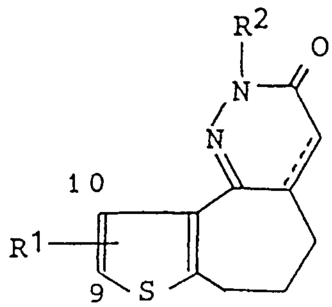

| No. | $R^1$ | $R^2$ | ---- bond |
|---|---|---|---|
| 48 | 9-COOH | 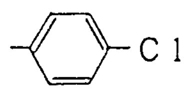 | single |
| 49 | $9-\underset{OH}{C}HCH_3$ | 〃 | 〃 |
| 50 | $9-COCH_2OCOCH_3$ | 〃 | 〃 |
| 51 | $9-CH_2CH_2OCOCH_3$ | 〃 | 〃 |
| 52 | $9-COOCH_3$ | 〃 | 〃 |
| 53 | 〃 | 〃 | double |
| 54 | $9-COCH_3$ | 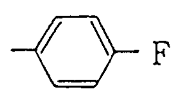 | single |
| 55 | $9-COCH_2Cl$ | 〃 | 〃 |
| 56 | 9-COO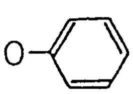 | 〃 | 〃 |
| 57 | $9-CH_2OCOCH_3$ | 〃 | 〃 |

Table 5

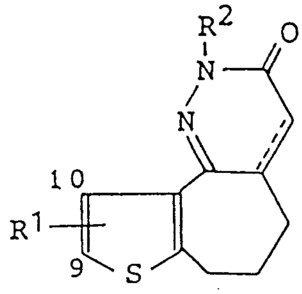

| No. | $R^1$ | $R^2$ | ---- bond |
|---|---|---|---|
| 58 | 9-CO 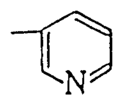 | 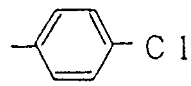 | single |
| 59 | 〃 | 〃 | double |
| 60 | $9-CH_2OCO$ 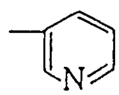 | 〃 | single |
| 61 | $9-COCH_2OCOCH_3$ | 〃 | 〃 |
| 62 | $9-COCH_2OCOCH_2$ 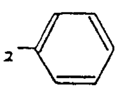 | 〃 | 〃 |
| 63 | $9-COCH_2$ 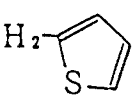 | 〃 | 〃 |
| 64 | $9-COCH_2OCOCH_2$ 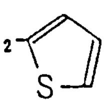 | 〃 | 〃 |
| 65 | 9-CO 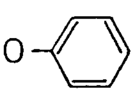 | 〃 | 〃 |
| 66 | 〃 | 〃 | double |
| 67 | 9-CO 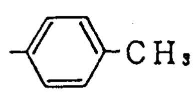 | 〃 | single |

Table 6

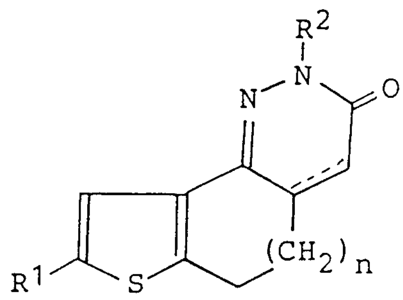

| No. | $R^1$ | $R^2$ | n | ---- bond |
|---|---|---|---|---|
| 68 | $-CH_2OH$ | 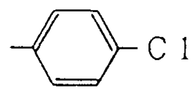 | 2 | double |
| 69 | $-\underset{OH}{\underset{\mid}{C}}HCH_3$ | 〃 | 1 | single |
| 70 | 〃 | 〃 | 1 | double |
| 71 | $-COCH_3$ | 〃 | 1 | 〃 |
| 72 | $-CHO$ | 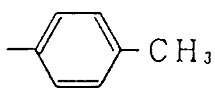 | 1 | 〃 |
| 73 | $-CH=NOH$ | 〃 | 1 | 〃 |
| 74 | $-CN$ | 〃 | 1 | 〃 |
| 75 | $-COCH_2OH$ | 〃 | 1 | single |
| 76 | $-CH_2CH_2OCH_3$ | 〃 | 1 | 〃 |

Formulation Example

Tablets containing 10 mg of a compound of the formula (I) are prepared in accordance with the following formulation:

| | |
|---|---|
| Compound of formula (I) | 10.0 mg |
| Lactose | 58.5 mg |
| Corn starch | 25.0 mg |
| Crystalline cellulose | 20.0 mg |
| Polyvinylpyrrolidone K-30 | 2.0 mg |
| Talc | 4.0 mg |
| Magnesium stearate | 0.5 mg |
| | 120.0 mg |

The compound of the formula (I) is pulverized by an atomizer into fine powders below 10 μ in average particle diameter, which are admixed with lactose, corn starch and crystalline cellulose sufficiently in a kneading machine, and further kneaded with polyvinylpyrrolidone paste. The kneaded mixture is passed through a sieve of 200 mesh, dried at 50 °C and passed through a sieve of 24 mesh. Talc and magnesium stearate are mixed therewith and the mixture is compressed into 120.0 mg tablets with a punch of 8mm in diameter. These tablets are, if desired, subjected to sugar-coating or film-coating.

While the present invention has been adequately and sufficiently described in the foregoing specification including examples, the description can be changed or modified within the spirit and scope of this invention.

## Claims

**1.** A fused pyridazine compound of the formula:

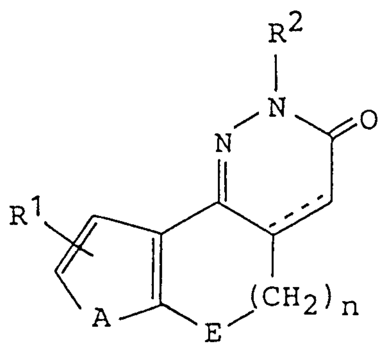

(I)

wherein:

$R^1$ is carboxyl, formyl, cyano, hydroxyiminomethyl, acyl, alkoxycarbonyl, aralkyloxycarbonyl, heteroarylalkyloxycarbonyl, aryloxycarbonyl, heteroaryloxycarbonyl, alkoxyalkyl, arylalkoxyalkyl, aryloxyalkyl, heteroaryloxyalkyl, acyloxyalkyl, hydroxyalkyl, acyloxyalkanoyl, alkoxyalkanoyl, hydroxyalkanoyl, aryloxyalkanoyl, haloalkanoyl, aralkyloxyalkanoyl, heteroarylalkyloxyalkanoyl or heteroaryloxyalkanoyl;

$R^2$ is hydrogen, alkyl having 1 to 8 carbon atoms, hydroxyalkyl, alkanoyloxyalkyl, aryl, aralkyl, heteroaryl, or aryl, aralkyl or heteroaryl having at least one substituent selected from the group consisting of halogen, hydroxy, amino, nitro, cyano, alkyl having 1 to 4 carbon atoms, alkoxy having 1 to 4 carbon atoms, alkanoylamino having 2 to 5 carbon atoms, haloalkyl, acyloxy, alkoxycarbonyl having 2 to 5 carbon atoms and carboxyl on the aromatic ring;

A is sulphur atom or $-CH=CH-$;

E is $-CH_2-$ or $-S(O)_m-$ (m is 0, 1 or 2);

n is 1 or 2;

the bond represented by full line and dotted line is a single bond or a double bond;

providing that when A is sulphur atom, E is $-CH_2-$, and when A is $-CH=CH-$, E is $-S(O)_m-$ (m is 0, 1 or 2) and n is 2.

**2.** The fused pyridazine compound according to Claim 1, wherein $R^2$ is aryl having at least one substituent selected from the group consisting of halogen, alkyl having 1 to 4 carbon atoms and alkoxy having 1 to 4 carbon atoms on the aromatic ring.

**3.** The fused pyridazine compound according to Claim 1, wherein A is sulphur atom, and E is $-CH_2-$.

**4.** The fused pyridazine compound according to Claim 1, wherein A is -CH = CH-, E is $-S(O)_m-$ (m is 0, 1 or 2) and n is 2.

**5.** The fused pyridazine compound according to Claim 1, which is selected from the group consisting of:
8-acetyl-2-(4-chlorophenyl)-4,4a,5,6-tetrahydrothieno[2,3-h]cinnolin-3(2H)-one,
2-(4-chlorophenyl)-4,4a,5,6-tetrahydro-8-propionylthieno[2,3-h]cinnolin-3(2H)-one,
8-butyryl-2-(4-chlorophenyl)-4,4a,5,6-tetrahydrothieno[2,3-h]cinnolin-3(2H)-one,
8-benzoyl-2-(4-chlorophenyl)-4,4a,5,6-tetrahydrothieno[2,3-h]cinnolin-3(2H)-one,
8-acetyl-4,4a,5,6-tetrahydro-2-(4-methylphenyl)thieno[2,3-h]cinnolin-3(2H)-one,
9-acetyl-2-(4-chlorophenyl)-2,4,4a,5,6,7-hexahydro-3H-thieno[2',3':6,7]cyclohepta[1,2-c]pyridazin-3-one,
9-acetyl-2-(4-chlorophenyl)-2,5,6,7-tetrahydro-3H-thieno[2',3':6,7]cyclohepta[1,2-c]pyridazin-3-one,
2-(4-chlorophenyl)-9-formyl-2,5,6,7-tetrahydro-3H-thieno[2',3':6,7]cyclohepta[1,2-c]pyridazin-3-one,
8-(1-hydroxyethyl)-4,4a,5,6-tetrahydro-2-(4-methylphenyl)thieno[2,3-h]cinnolin-3(2H)-one,
8-acetyl-5,6-dihydro-2-(4-methylphenyl)thieno[2,3-h]cinnolin-3(2H)-one,
8-(1-hydroxyethyl)-5,6-dihydro-2-(4-methylphenyl)thieno[2,3-h]cinnolin-3(2H)-one,
8-chloroacetyl-4,4a,5,6-tetrahydro-2-(4-methylphenyl)thieno[2,3-h]cinnolin-3(2H)-one,
8-acetoxyacetyl-4,4a,5,6-tetrahydro-2-(4-methylphenyl)thieno[2,3-h]cinnolin-3(2H)-one,
8-(2-acetoxyethyl)-4,4a,5,6-tetrahydro-2-(4-methylphenyl)thieno[2,3-h]cinnolin-3(2H)-one,
4,4a,5,6-tetrahydro-8-(2-hydroxyethyl)-2-(4-methylphenyl)thieno[2,3-h]cinnolin-3(2H)-one,
10-acetyl-2-(4-chlorophenyl)-5,6-dihydro-[1]benzothiepino[5,4-c]pyridazin-3(2H)-one,
2-(4-chlorophenyl)-10-(1-hydroxyethyl)-5,6-dihydro-[1]benzothiepino[5,4-c]pyridazin-3(2H)-one,
10-acetyl-2-(4-chlorophenyl)-5,6-dihydro-[1]benzothiepino[5,4-c]pyridazin-3(2H)-one 7,7-dioxide,
2-(4-chlorophenyl)-10-(1-hydroxyethyl)-5,6-dihydro-[1]benzothiepino[5,4-c]pyridazin-3(2H)-one 7-oxide,
2-(4-chlorophenyl)-10-(1-formyloxyethyl)-5,6-dihydro-[1]benzothiepino[5,4-c]pyridazin-3(2H)-one 7-oxide,
2-(4-chlorophenyl)-5,6-dihydro-10-propionyl-[1]benzothiepino[5,4-c]pyridazin-3(2H)one,
10-butyryl-2-(4-chlorophenyl)-5,6-dihydro-[1]benzothiepino[5,4-c]pyridazin-3(2H)-one,
2-(4-chlorophenyl)-10-(1-hydroxybutyl)-5,6-dihydro-[1]benzothiepino[5,4-c]pyridazin-3(2H)-one,
2-(4-chlorophenyl)-10-(1-hydroxyethyl)-5,6-dihydro-[1]benzothiepino[5,4-c]pyridazin-3(2H)-one 7,7-dioxide,
10-acetyl-2-(4-chlorophenyl)-5,6-dihydro-[1]benzothiepino[5,4-c]pyridazin-3(2H)-one 7-oxide,
10-chloroacetyl-2-(4-chlorophenyl)-5,6-dihydro-[1]benzothiepino[5,4-c]pyridazin-3(2H)-one,
methyl 2-(4-chlorophenyl)-2,3,5,6-tetrahydro-3-oxo-[1]benzothiepino[5,4-c]pyridazine-10-carboxylate, and
2-(4-chlorophenyl)-2,3,5,6-tetrahydro-3-oxo-[1]benzothiepino[5,4-c]pyridazine-10-carboxylic acid.

**6.** The fused pyridazine compound according to Claim 1, wherein the compound of formula (I) is 9-acetyl-2-(4-chlorophenyl)-2,4,4a,5,6,7-hexahydro-3H-thieno[2',3':6,7]cyclohepta[1,2-c]pyridazin-3-one.

**7.** A pharmaceutical composition comprising a therapeutically effective amount of the compound of Claim 1 and pharmaceutically acceptable additives.

**8.** An anxiolytic agent containing a comound of Claim 1 as an effective ingredient.

# INTERNATIONAL SEARCH REPORT

International Application No PCT/JP92/01067

**I. CLASSIFICATION OF SUBJECT MATTER** (if several classification symbols apply, indicate all) [6]

According to International Patent Classification (IPC) or to both National Classification and IPC

Int. $Cl^5$ C07D495/04, A61K31/50

**II. FIELDS SEARCHED**

Minimum Documentation Searched [7]

| Classification System | Classification Symbols |
|---|---|
| IPC | C07D495/04, A61K31/495-31/50 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| A | JP, A, 64-6278 (Yoshitomi Pharmaceutical Co., Ltd.), January 10, 1989 (10. 01. 89), (Family: none) | 1-3, 5, 7, 8 |
| A | JP, A, 2-167285 (Yoshitomi Pharmaceutical Co., Ltd.), June 27, 1990 (27. 06. 90), & WO, A1, 9003380 & EP, A1, 394471 | 1, 2, 5, 6-8 |
| A | JP, A, 1-250383 (Yoshitomi Pharmaceutical Co., Ltd.), October 5, 1989 (05. 10. 89), & EP, A1, 281045 & US, A, 4849421 | 1, 2, 4, 5, 7, 8 |
| A | JP, A, 2-69481 (Yoshitomi Pharmaceutical Co., Ltd.), March 8, 1990 (08. 03. 90), (Family: none) | 1, 2, 4, 5, 7, 8 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| November 10, 1992 (10. 11. 92) | December 1, 1992 (01. 12. 92) |
| **International Searching Authority** | **Signature of Authorized Officer** |
| Japanese Patent Office | |